# EUROPEAN PATENT APPLICATION

(11) **EP 2 599 789 A1**
(43) Date of publication of application: **05.06.2013**
(21) Application number: 11191486.7
(22) Date of filing: 01.12.2011
(51) Int. Cl.: C07K 14/435

(54) **Marker and method for diagnosing diarrhea syndrome**

(71) Applicant: Bergen Teknologioverføring AS, 5006 Bergen (NO)
(72) Inventor: Knappskog, Per, 5006 Bergen (NO); Fiskerstrand, Torunn, 5006 Bergen (NO)
(74) Representative: Kröncke, Rolf

(57) **Abstract**

The present invention relates in a first aspect to new nucleic acid molecules and peptides identifying and diagnosing diarrhea syndrome including chronic diarrhea and bowel diseases. In addition, methods are provided for identifying or diagnosing a potential risk for developing or having a diarrhea syndrome comprising identifying in a sample from a subject the presence or absence of a mutation at position 2519 of the gene encoding GC-C. Moreover, a kit is provided allowing identification or diagnosing a diarrhea syndrome including probes allowing the detection of mutations at position 2519 of the nucleic acid sequence encoding GC-C or for detecting a mutation at position 840 of GC-C. Moreover, a method for screening and/or identifying candidate molecules useful in prophylactically or therapeutically treating said diseases is provided. Finally, the present invention relates to metforminand/or a pharmaceutically acceptable salts thereof for use in the prophylactic or therapeutic treatment of diarrhea syndrome.

SEQUENCE LISTING

<110>  Bergen Teknologioverforing AS

<120>  Marker and method for diagnosing diarrhea syndrome

<130>  4200-019-EP-1

<160>  13

<170>  PatentIn version 3.5

<210>  1
<211>  48
<212>  DNA
<213>  homo sapiens

<400>  1
ttcactacta tctgcaaata catcaccccc atggaagtgg tggacatg                48


<210>  2
<211>  3713
<212>  DNA
<213>  homo sapiens


<220>
<221>  CDS
<222>  (1)..(3222)

<400>  2
atg aag acg ttg ctg ttg gac ttg gct ttg tgg tca ctg ctc ttc cag      48
Met Lys Thr Leu Leu Leu Asp Leu Ala Leu Trp Ser Leu Leu Phe Gln
1               5                   10                  15

ccc ggg tgg ctg tcc ttt agt tcc cag gtg agt cag aac tgc cac aat      96
Pro Gly Trp Leu Ser Phe Ser Ser Gln Val Ser Gln Asn Cys His Asn
            20                  25                  30

ggc agc tat gaa atc agc gtc ctg atg atg ggc aac tca gcc ttt gca     144
Gly Ser Tyr Glu Ile Ser Val Leu Met Met Gly Asn Ser Ala Phe Ala
        35                  40                  45

gag ccc ctg aaa aac ttg gaa gat gcg gtg aat gag ggg ctg gaa ata     192
Glu Pro Leu Lys Asn Leu Glu Asp Ala Val Asn Glu Gly Leu Glu Ile
    50                  55                  60

gtg aga gga cgt ctg caa aat gct ggc cta aat gtg act gtg aac gct     240
Val Arg Gly Arg Leu Gln Asn Ala Gly Leu Asn Val Thr Val Asn Ala
65                  70                  75                  80

act ttc atg tat tcg gat ggt ctg att cat aac tca ggc gac tgc cgg     288
Thr Phe Met Tyr Ser Asp Gly Leu Ile His Asn Ser Gly Asp Cys Arg
                85                  90                  95

agt agc acc tgt gaa ggc ctc gac cta ctc agg aaa att tca aat gca     336
Ser Ser Thr Cys Glu Gly Leu Asp Leu Leu Arg Lys Ile Ser Asn Ala
            100                 105                 110

caa cgg atg ggc tgt gtc ctc ata ggg ccc tca tgt aca tac tcc acc     384
Gln Arg Met Gly Cys Val Leu Ile Gly Pro Ser Cys Thr Tyr Ser Thr
        115                 120                 125

ttc cag atg tac ctt gac aca gaa ttg agc tac ccc atg atc tca gct     432
Phe Gln Met Tyr Leu Asp Thr Glu Leu Ser Tyr Pro Met Ile Ser Ala
    130                 135                 140

gga agt ttt gga ttg tca tgt gac tat aaa gaa acc tta acc agg ctg 480
Gly Ser Phe Gly Leu Ser Cys Asp Tyr Lys Glu Thr Leu Thr Arg Leu
145                 150                 155                 160

atg tct cca gct aga aag ttg atg tac ttc ttg gtt aac ttt tgg aaa 528
Met Ser Pro Ala Arg Lys Leu Met Tyr Phe Leu Val Asn Phe Trp Lys
                165                 170                 175

acc aac gat ctg ccc ttc aaa act tat tcc tgg agc act tcg tat gtt 576
Thr Asn Asp Leu Pro Phe Lys Thr Tyr Ser Trp Ser Thr Ser Tyr Val
            180                 185                 190

tac aag aat ggt aca gaa act gag gac tgt ttc tgg tac ctt aat gct 624
Tyr Lys Asn Gly Thr Glu Thr Glu Asp Cys Phe Trp Tyr Leu Asn Ala
        195                 200                 205

ctg gag gct agc gtt tcc tat ttc tcc cac gaa ctc ggc ttt aag gtg 672
Leu Glu Ala Ser Val Ser Tyr Phe Ser His Glu Leu Gly Phe Lys Val
    210                 215                 220

gtg tta aga caa gat aag gag ttt cag gat atc tta atg gac cac aac 720
Val Leu Arg Gln Asp Lys Glu Phe Gln Asp Ile Leu Met Asp His Asn
225                 230                 235                 240

agg aaa agc aat gtg att att atg tgt ggt ggt cca gag ttc ctc tac 768
Arg Lys Ser Asn Val Ile Ile Met Cys Gly Gly Pro Glu Phe Leu Tyr
                245                 250                 255

aag ctg aag ggt gac cga gca gtg gct gaa gac att gtc att att cta 816
Lys Leu Lys Gly Asp Arg Ala Val Ala Glu Asp Ile Val Ile Ile Leu
            260                 265                 270

gtg gat ctt ttc aat gac cag tac ttt gag gac aat gtc aca gcc cct 864
Val Asp Leu Phe Asn Asp Gln Tyr Phe Glu Asp Asn Val Thr Ala Pro
        275                 280                 285

gac tat atg aaa aat gtc ctt gtt ctg acg ctg tct cct ggg aat tcc 912
Asp Tyr Met Lys Asn Val Leu Val Leu Thr Leu Ser Pro Gly Asn Ser
    290                 295                 300

ctt cta aat agc tct ttc tcc agg aat cta tca cca aca aaa cga gac 960
Leu Leu Asn Ser Ser Phe Ser Arg Asn Leu Ser Pro Thr Lys Arg Asp
305                 310                 315                 320

ttt gct ctt gcc tat ttg aat gga atc ctg ctc ttt gga cat atg ctg 1008
Phe Ala Leu Ala Tyr Leu Asn Gly Ile Leu Leu Phe Gly His Met Leu
                325                 330                 335

aag ata ttt ctt gaa aat gga gaa aat att acc acc ccc aaa ttt gct 1056
Lys Ile Phe Leu Glu Asn Gly Glu Asn Ile Thr Thr Pro Lys Phe Ala
            340                 345                 350

cat gct ttc agg aat ctc act ttt gaa ggg tat gac ggt cca gtg acc 1104
His Ala Phe Arg Asn Leu Thr Phe Glu Gly Tyr Asp Gly Pro Val Thr
        355                 360                 365

ttg gat gac tgg ggg gat gtt gac agt acc atg gtg ctt ctg tat acc 1152
Leu Asp Asp Trp Gly Asp Val Asp Ser Thr Met Val Leu Leu Tyr Thr
    370                 375                 380

tct gtg gac acc aag aaa tac aag gtt ctt ttg acc tat gat acc cac 1200
Ser Val Asp Thr Lys Lys Tyr Lys Val Leu Leu Thr Tyr Asp Thr His
385                 390                 395                 400

gta aat aag acc tat cct gtg gat atg agc ccc aca ttc act tgg aag 1248
Val Asn Lys Thr Tyr Pro Val Asp Met Ser Pro Thr Phe Thr Trp Lys
                405                 410                 415

aac tct aaa ctt cct aat gat att aca ggc cgg ggc cct cag atc ctg          1296
Asn Ser Lys Leu Pro Asn Asp Ile Thr Gly Arg Gly Pro Gln Ile Leu
            420                 425                 430

atg att gca gtc ttc acc ctc act gga gct gtg gtg ctg ctc ctg ctc          1344
Met Ile Ala Val Phe Thr Leu Thr Gly Ala Val Val Leu Leu Leu Leu
        435                 440                 445

gtc gct ctc ctg atg ctc aga aaa tat aga aaa gat tat gaa ctt cgt          1392
Val Ala Leu Leu Met Leu Arg Lys Tyr Arg Lys Asp Tyr Glu Leu Arg
    450                 455                 460

cag aaa aaa tgg tcc cac att cct cct gaa aat atc ttt cct ctg gag          1440
Gln Lys Lys Trp Ser His Ile Pro Pro Glu Asn Ile Phe Pro Leu Glu
465                 470                 475                 480

acc aat gag acc aat cat gtt agc ctc aag atc gat gat gac aaa aga          1488
Thr Asn Glu Thr Asn His Val Ser Leu Lys Ile Asp Asp Asp Lys Arg
                485                 490                 495

cga gat aca atc cag aga cta cga cag tgc aaa tac gac aaa aag cga          1536
Arg Asp Thr Ile Gln Arg Leu Arg Gln Cys Lys Tyr Asp Lys Lys Arg
            500                 505                 510

gtg att ctc aaa gat ctc aag cac aat gat ggt aat ttc act gaa aaa          1584
Val Ile Leu Lys Asp Leu Lys His Asn Asp Gly Asn Phe Thr Glu Lys
        515                 520                 525

cag aag ata gaa ttg aac aag ttg ctt cag att gac tat tac aac ctg          1632
Gln Lys Ile Glu Leu Asn Lys Leu Leu Gln Ile Asp Tyr Tyr Asn Leu
    530                 535                 540

acc aag ttc tac ggc aca gtg aaa ctt gat acc atg atc ttc ggg gtg          1680
Thr Lys Phe Tyr Gly Thr Val Lys Leu Asp Thr Met Ile Phe Gly Val
545                 550                 555                 560

ata gaa tac tgt gag aga gga tcc ctc cgg gaa gtt tta aat gac aca          1728
Ile Glu Tyr Cys Glu Arg Gly Ser Leu Arg Glu Val Leu Asn Asp Thr
                565                 570                 575

att tcc tac cct gat ggc aca ttc atg gat tgg gag ttt aag atc tct          1776
Ile Ser Tyr Pro Asp Gly Thr Phe Met Asp Trp Glu Phe Lys Ile Ser
            580                 585                 590

gtc ttg tat gac att gct aag gga atg tca tat ctg cac tcc agt aag          1824
Val Leu Tyr Asp Ile Ala Lys Gly Met Ser Tyr Leu His Ser Ser Lys
        595                 600                 605

aca gaa gtc cat ggt cgt ctg aaa tct acc aac tgc gta gtg gac agt          1872
Thr Glu Val His Gly Arg Leu Lys Ser Thr Asn Cys Val Val Asp Ser
    610                 615                 620

aga atg gtg gtg aag atc act gat ttt ggc tgc aat tcc att tta cct          1920
Arg Met Val Val Lys Ile Thr Asp Phe Gly Cys Asn Ser Ile Leu Pro
625                 630                 635                 640

cca aaa aag gac ctg tgg aca gct cca gag cac ctc cgc caa gcc aac          1968
Pro Lys Lys Asp Leu Trp Thr Ala Pro Glu His Leu Arg Gln Ala Asn
                645                 650                 655

atc tct cag aaa gga gat gtg tac agc tat ggg atc atc gca cag gag          2016
Ile Ser Gln Lys Gly Asp Val Tyr Ser Tyr Gly Ile Ile Ala Gln Glu
            660                 665                 670

atc atc ctg cgg aaa gaa acc ttc tac act ttg agc tgt cgg gac cgg          2064
Ile Ile Leu Arg Lys Glu Thr Phe Tyr Thr Leu Ser Cys Arg Asp Arg
        675                 680                 685

aat gag aag att ttc aga gtg gaa aat tcc aat gga atg aaa ccc ttc 2112
Asn Glu Lys Ile Phe Arg Val Glu Asn Ser Asn Gly Met Lys Pro Phe
    690                 695                 700

cgc cca gat tta ttc ttg gaa aca gca gag gaa aaa gag cta gaa gtg 2160
Arg Pro Asp Leu Phe Leu Glu Thr Ala Glu Glu Lys Glu Leu Glu Val
705                 710                 715                 720

tac cta ctt gta aaa aac tgt tgg gag gaa gat cca gaa aag aga cca 2208
Tyr Leu Leu Val Lys Asn Cys Trp Glu Glu Asp Pro Glu Lys Arg Pro
                725                 730                 735

gat ttc aaa aaa att gag act aca ctt gcc aag ata ttt gga ctt ttt 2256
Asp Phe Lys Lys Ile Glu Thr Thr Leu Ala Lys Ile Phe Gly Leu Phe
            740                 745                 750

cat gac caa aaa aat gaa agc tat atg gat acc ttg atc cga cgt cta 2304
His Asp Gln Lys Asn Glu Ser Tyr Met Asp Thr Leu Ile Arg Arg Leu
        755                 760                 765

cag cta tat tct cga aac ctg gaa cat ctg gta gag gaa agg aca cag 2352
Gln Leu Tyr Ser Arg Asn Leu Glu His Leu Val Glu Glu Arg Thr Gln
    770                 775                 780

ctg tac aag gca gag agg gac agg gct gac aga ctt aac ttt atg ttg 2400
Leu Tyr Lys Ala Glu Arg Asp Arg Ala Asp Arg Leu Asn Phe Met Leu
785                 790                 795                 800

ctt cca agg cta gtg gta aag tct ctg aag gag aaa ggc ttt gtg gag 2448
Leu Pro Arg Leu Val Val Lys Ser Leu Lys Glu Lys Gly Phe Val Glu
                805                 810                 815

ccg gaa cta tat gag gaa gtt aca atc tac ttc agt gac att gta ggt 2496
Pro Glu Leu Tyr Glu Glu Val Thr Ile Tyr Phe Ser Asp Ile Val Gly
            820                 825                 830

ttc act act atc tgc aaa tac atc acc ccc atg gaa gtg gtg gac atg 2544
Phe Thr Thr Ile Cys Lys Tyr Ile Thr Pro Met Glu Val Val Asp Met
        835                 840                 845

ctt aat gac atc tat aag agt ttt gac cac att gtt gat cat cat gat 2592
Leu Asn Asp Ile Tyr Lys Ser Phe Asp His Ile Val Asp His His Asp
    850                 855                 860

gtc tac aag gtg gaa acc atc ggt gat gcg tac atg gtg gct agt ggt 2640
Val Tyr Lys Val Glu Thr Ile Gly Asp Ala Tyr Met Val Ala Ser Gly
865                 870                 875                 880

ttg cct aag aga aat ggc aat cgg cat gca ata gac att gcc aag atg 2688
Leu Pro Lys Arg Asn Gly Asn Arg His Ala Ile Asp Ile Ala Lys Met
                885                 890                 895

gcc ttg gaa atc ctc agc ttc atg ggg acc ttt gag ctg gag cat ctt 2736
Ala Leu Glu Ile Leu Ser Phe Met Gly Thr Phe Glu Leu Glu His Leu
            900                 905                 910

cct ggc ctc cca ata tgg att cgc att gga gtt cac tct ggt ccc tgt 2784
Pro Gly Leu Pro Ile Trp Ile Arg Ile Gly Val His Ser Gly Pro Cys
        915                 920                 925

gct gct gga gtt gtg gga atc aag atg cct cgt tat tgt cta ttt gga 2832
Ala Ala Gly Val Val Gly Ile Lys Met Pro Arg Tyr Cys Leu Phe Gly
    930                 935                 940

gat acg gtc aac aca gcc tct agg atg gaa tcc act ggc ctc cct ttg 2880
Asp Thr Val Asn Thr Ala Ser Arg Met Glu Ser Thr Gly Leu Pro Leu
945                 950                 955                 960

aga att cac gtg agt ggc tcc acc ata gcc atc ctg aag aga act gag     2928
Arg Ile His Val Ser Gly Ser Thr Ile Ala Ile Leu Lys Arg Thr Glu
                965                 970                 975

tgc cag ttc ctt tat gaa gtg aga gga gaa aca tac tta aag gga aga     2976
Cys Gln Phe Leu Tyr Glu Val Arg Gly Glu Thr Tyr Leu Lys Gly Arg
            980                 985                 990

gga aat gag act acc tac tgg ctg  act ggg atg aag gac  cag aaa ttc     3024
Gly Asn Glu Thr Thr Tyr Trp Leu  Thr Gly Met Lys Asp  Gln Lys Phe
        995                 1000                 1005

aac ctg  cca acc cct cct act  gtg gag aat caa cag  cgt ttg caa     3069
Asn Leu  Pro Thr Pro Pro Thr  Val Glu Asn Gln Gln  Arg Leu Gln
    1010                 1015                 1020

gca gaa  ttt tca gac atg att  gcc aac tct tta cag  aaa aga cag     3114
Ala Glu  Phe Ser Asp Met Ile  Ala Asn Ser Leu Gln  Lys Arg Gln
    1025                 1030                 1035

gca gca  ggg ata aga agc caa  aaa ccc aga cgg gta  gcc agc tat     3159
Ala Ala  Gly Ile Arg Ser Gln  Lys Pro Arg Arg Val  Ala Ser Tyr
    1040                 1045                 1050

aaa aaa  ggc act ctg gaa tac  ttg cag ctg aat acc  aca gac aag     3204
Lys Lys  Gly Thr Leu Glu Tyr  Leu Gln Leu Asn Thr  Thr Asp Lys
    1055                 1060                 1065

gag agc  acc tat ttt taa acctaaatga ggtataagga ctcacacaaa     3252
Glu Ser  Thr Tyr Phe
    1070

ttaaaataca gctgcactga ggcagcgacc tcaagtgtcc tgaaagctta cattttcctg     3312

agacctcaat gaagcagaaa tgtacttagg cttggctgcc ctgtctggaa catggacttt     3372

cttgcatgaa tcagatgtgt gttctcagtg aaataactac cttccactct ggaaccttat     3432

tccagcagtt gttccaggga gcttctacct ggaaaagaaa agaaatgaat agactatcta     3492

gaacttgaga agattttatt cttatttcat ttattttttg tttgtttatt tttatcgttt     3552

ttgtttactg gctttccttc tgtattcata agatttttta aattgtcata attatatttt     3612

aaatacccat cttcattaaa gtatatttaa ctcataattt ttgcagaaaa tatgctatat     3672

attaggcaag aataaaagct aaaggtttcc caaaaaaaaa a                         3713


<210>  3
<211>  1073
<212>  PRT
<213>  homo sapiens

<400>  3

Met Lys Thr Leu Leu Leu Asp Leu Ala Leu Trp Ser Leu Leu Phe Gln
1               5                   10                  15


Pro Gly Trp Leu Ser Phe Ser Ser Gln Val Ser Gln Asn Cys His Asn
            20                  25                  30


Gly Ser Tyr Glu Ile Ser Val Leu Met Met Gly Asn Ser Ala Phe Ala
        35                  40                  45

Glu Pro Leu Lys Asn Leu Glu Asp Ala Val Asn Glu Gly Leu Glu Ile
    50                  55                  60

Val Arg Gly Arg Leu Gln Asn Ala Gly Leu Asn Val Thr Val Asn Ala
65                  70                  75                  80

Thr Phe Met Tyr Ser Asp Gly Leu Ile His Asn Ser Gly Asp Cys Arg
                85                  90                  95

Ser Ser Thr Cys Glu Gly Leu Asp Leu Leu Arg Lys Ile Ser Asn Ala
            100                 105                 110

Gln Arg Met Gly Cys Val Leu Ile Gly Pro Ser Cys Thr Tyr Ser Thr
        115                 120                 125

Phe Gln Met Tyr Leu Asp Thr Glu Leu Ser Tyr Pro Met Ile Ser Ala
    130                 135                 140

Gly Ser Phe Gly Leu Ser Cys Asp Tyr Lys Glu Thr Leu Thr Arg Leu
145                 150                 155                 160

Met Ser Pro Ala Arg Lys Leu Met Tyr Phe Leu Val Asn Phe Trp Lys
                165                 170                 175

Thr Asn Asp Leu Pro Phe Lys Thr Tyr Ser Trp Ser Thr Ser Tyr Val
            180                 185                 190

Tyr Lys Asn Gly Thr Glu Thr Glu Asp Cys Phe Trp Tyr Leu Asn Ala
        195                 200                 205

Leu Glu Ala Ser Val Ser Tyr Phe Ser His Glu Leu Gly Phe Lys Val
    210                 215                 220

Val Leu Arg Gln Asp Lys Glu Phe Gln Asp Ile Leu Met Asp His Asn
225                 230                 235                 240

Arg Lys Ser Asn Val Ile Ile Met Cys Gly Gly Pro Glu Phe Leu Tyr
                245                 250                 255

Lys Leu Lys Gly Asp Arg Ala Val Ala Glu Asp Ile Val Ile Ile Leu
            260                 265                 270

Val Asp Leu Phe Asn Asp Gln Tyr Phe Glu Asp Asn Val Thr Ala Pro
        275                 280                 285

Asp Tyr Met Lys Asn Val Leu Val Leu Thr Leu Ser Pro Gly Asn Ser
    290                 295                 300

Leu Leu Asn Ser Ser Phe Ser Arg Asn Leu Ser Pro Thr Lys Arg Asp
305                 310                 315                 320

Phe Ala Leu Ala Tyr Leu Asn Gly Ile Leu Leu Phe Gly His Met Leu
                325                 330                 335

Lys Ile Phe Leu Glu Asn Gly Glu Asn Ile Thr Thr Pro Lys Phe Ala
            340                 345                 350

His Ala Phe Arg Asn Leu Thr Phe Glu Gly Tyr Asp Gly Pro Val Thr
        355                 360                 365

Leu Asp Asp Trp Gly Asp Val Asp Ser Thr Met Val Leu Leu Tyr Thr
    370                 375                 380

Ser Val Asp Thr Lys Lys Tyr Lys Val Leu Leu Thr Tyr Asp Thr His
385                 390                 395                 400

Val Asn Lys Thr Tyr Pro Val Asp Met Ser Pro Thr Phe Thr Trp Lys
                405                 410                 415

Asn Ser Lys Leu Pro Asn Asp Ile Thr Gly Arg Gly Pro Gln Ile Leu
            420                 425                 430

Met Ile Ala Val Phe Thr Leu Thr Gly Ala Val Val Leu Leu Leu Leu
        435                 440                 445

Val Ala Leu Leu Met Leu Arg Lys Tyr Arg Lys Asp Tyr Glu Leu Arg
    450                 455                 460

Gln Lys Lys Trp Ser His Ile Pro Pro Glu Asn Ile Phe Pro Leu Glu
465                 470                 475                 480

Thr Asn Glu Thr Asn His Val Ser Leu Lys Ile Asp Asp Asp Lys Arg
                485                 490                 495

Arg Asp Thr Ile Gln Arg Leu Arg Gln Cys Lys Tyr Asp Lys Lys Arg
            500                 505                 510

Val Ile Leu Lys Asp Leu Lys His Asn Asp Gly Asn Phe Thr Glu Lys
        515                 520                 525

Gln Lys Ile Glu Leu Asn Lys Leu Leu Gln Ile Asp Tyr Tyr Asn Leu
    530                 535                 540

Thr Lys Phe Tyr Gly Thr Val Lys Leu Asp Thr Met Ile Phe Gly Val
545                 550                 555                 560

Ile Glu Tyr Cys Glu Arg Gly Ser Leu Arg Glu Val Leu Asn Asp Thr
                565                 570                 575

Ile Ser Tyr Pro Asp Gly Thr Phe Met Asp Trp Glu Phe Lys Ile Ser
            580                 585                 590

Val Leu Tyr Asp Ile Ala Lys Gly Met Ser Tyr Leu His Ser Ser Lys
        595                 600                 605

Thr Glu Val His Gly Arg Leu Lys Ser Thr Asn Cys Val Val Asp Ser
    610                 615                 620

Arg Met Val Val Lys Ile Thr Asp Phe Gly Cys Asn Ser Ile Leu Pro
625                 630                 635                 640

Pro Lys Lys Asp Leu Trp Thr Ala Pro Glu His Leu Arg Gln Ala Asn
                645                 650                 655

Ile Ser Gln Lys Gly Asp Val Tyr Ser Tyr Gly Ile Ile Ala Gln Glu
            660                 665                 670

Ile Ile Leu Arg Lys Glu Thr Phe Tyr Thr Leu Ser Cys Arg Asp Arg
        675                 680                 685

Asn Glu Lys Ile Phe Arg Val Glu Asn Ser Asn Gly Met Lys Pro Phe
    690                 695                 700

Arg Pro Asp Leu Phe Leu Glu Thr Ala Glu Glu Lys Glu Leu Glu Val
705                 710                 715                 720

Tyr Leu Leu Val Lys Asn Cys Trp Glu Glu Asp Pro Glu Lys Arg Pro
                725                 730                 735

Asp Phe Lys Lys Ile Glu Thr Thr Leu Ala Lys Ile Phe Gly Leu Phe
            740                 745                 750

His Asp Gln Lys Asn Glu Ser Tyr Met Asp Thr Leu Ile Arg Arg Leu
        755                 760                 765

Gln Leu Tyr Ser Arg Asn Leu Glu His Leu Val Glu Glu Arg Thr Gln
    770                 775                 780

Leu Tyr Lys Ala Glu Arg Asp Arg Ala Asp Arg Leu Asn Phe Met Leu
785                 790                 795                 800

Leu Pro Arg Leu Val Val Lys Ser Leu Lys Glu Lys Gly Phe Val Glu
                805                 810                 815

Pro Glu Leu Tyr Glu Glu Val Thr Ile Tyr Phe Ser Asp Ile Val Gly
            820                 825                 830

Phe Thr Thr Ile Cys Lys Tyr Ile Thr Pro Met Glu Val Val Asp Met
        835                 840                 845

Leu Asn Asp Ile Tyr Lys Ser Phe Asp His Ile Val Asp His His Asp
    850                 855                 860

Val Tyr Lys Val Glu Thr Ile Gly Asp Ala Tyr Met Val Ala Ser Gly
865                 870                 875                 880

Leu Pro Lys Arg Asn Gly Asn Arg His Ala Ile Asp Ile Ala Lys Met
                885                 890                 895

Ala Leu Glu Ile Leu Ser Phe Met Gly Thr Phe Glu Leu Glu His Leu
            900                 905                 910

Pro Gly Leu Pro Ile Trp Ile Arg Ile Gly Val His Ser Gly Pro Cys
        915                 920                 925

Ala Ala Gly Val Val Gly Ile Lys Met Pro Arg Tyr Cys Leu Phe Gly
    930                 935                 940

Asp Thr Val Asn Thr Ala Ser Arg Met Glu Ser Thr Gly Leu Pro Leu
945                 950                 955                 960

Arg Ile His Val Ser Gly Ser Thr Ile Ala Ile Leu Lys Arg Thr Glu
                965                 970                 975

Cys Gln Phe Leu Tyr Glu Val Arg Gly Glu Thr Tyr Leu Lys Gly Arg
            980                 985                 990

Gly Asn Glu Thr Thr Tyr Trp Leu  Thr Gly Met Lys Asp  Gln Lys Phe
        995                 1000                 1005

Asn Leu  Pro Thr Pro Pro Thr  Val Glu Asn Gln Gln  Arg Leu Gln
    1010                 1015                 1020

Ala Glu  Phe Ser Asp Met Ile  Ala Asn Ser Leu Gln  Lys Arg Gln
    1025                 1030                 1035

Ala Ala  Gly Ile Arg Ser Gln  Lys Pro Arg Arg Val  Ala Ser Tyr
    1040                 1045                 1050

Lys Lys  Gly Thr Leu Glu Tyr  Leu Gln Leu Asn Thr  Thr Asp Lys
    1055                 1060                 1065

Glu Ser  Thr Tyr Phe
    1070

<210>  4
<211>  3713
<212>  DNA
<213>  homo sapiens

<220>
<221>  CDS
<222>  (1)..(3222)

<400>  4
atg aag acg ttg ctg ttg gac ttg gct ttg tgg tca ctg ctc ttc cag          48
ccc ggg tgg ctg tcc ttt agt tcc cag gtg agt cag aac tgc cac aat      96
ggc agc tat gaa atc agc gtc ctg atg atg ggc aac tca gcc ttt gca      144
gag ccc ctg aaa aac ttg gaa gat gcg gtg aat gag ggg ctg gaa ata      192
gtg aga gga cgt ctg caa aat gct ggc cta aat gtg act gtg aac gct      240
act ttc atg tat tcg gat ggt ctg att cat aac tca ggc gac tgc cgg      288
agt agc acc tgt gaa ggc ctc gac cta ctc agg aaa att tca aat gca      336
caa cgg atg ggc tgt gtc ctc ata ggg ccc tca tgt aca tac tcc acc      384
ttc cag atg tac ctt gac aca gaa ttg agc tac ccc atg atc tca gct      432
gga agt ttt gga ttg tca tgt gac tat aaa gaa acc tta acc agg ctg      480
atg tct cca gct aga aag ttg atg tac ttc ttg gtt aac ttt tgg aaa      528
acc aac gat ctg ccc ttc aaa act tat tcc tgg agc act tcg tat gtt      576
tac aag aat ggt aca gaa act gag gac tgt ttc tgg tac ctt aat gct      624
ctg gag gct agc gtt tcc tat ttc tcc cac gaa ctc ggc ttt aag gtg      672
gtg tta aga caa gat aag gag ttt cag gat atc tta atg gac cac aac      720
agg aaa agc aat gtg att att atg tgt ggt ggt cca gag ttc ctc tac      768
aag ctg aag ggt gac cga gca gtg gct gaa gac att gtc att att cta      816
gtg gat ctt ttc aat gac cag tac ttt gag gac aat gtc aca gcc cct      864
gac tat atg aaa aat gtc ctt gtt ctg acg ctg tct cct ggg aat tcc      912
ctt cta aat agc tct ttc tcc agg aat cta tca cca aca aaa cga gac      960
ttt gct ctt gcc tat ttg aat gga atc ctg ctc ttt gga cat atg ctg     1008
aag ata ttt ctt gaa aat gga gaa aat att acc acc ccc aaa ttt gct     1056
cat gct ttc agg aat ctc act ttt gaa ggg tat gac ggt cca gtg acc     1104
ttg gat gac tgg ggg gat gtt gac agt acc atg gtg ctt ctg tat acc     1152
tct gtg gac acc aag aaa tac aag gtt ctt ttg acc tat gat acc cac     1200
gta aat aag acc tat cct gtg gat atg agc ccc aca ttc act tgg aag     1248
aac tct aaa ctt cct aat gat att aca ggc cgg ggc cct cag atc ctg     1296
atg att gca gtc ttc acc ctc act gga gct gtg gtg ctg ctc ctg ctc     1344
gtc gct ctc ctg atg ctc aga aaa tat aga aaa gat tat gaa ctt cgt     1392
cag aaa aaa tgg tcc cac att cct cct gaa aat atc ttt cct ctg gag     1440
acc aat gag acc aat cat gtt agc ctc aag atc gat gat gac aaa aga     1488
cga gat aca atc cag aga cta cga cag tgc aaa tac gac aaa aag cga     1536
gtg att ctc aaa gat ctc aag cac aat gat ggt aat ttc act gaa aaa     1584
cag aag ata gaa ttg aac aag ttg ctt cag att gac tat tac aac ctg     1632
acc aag ttc tac ggc aca gtg aaa ctt gat acc atg atc ttc ggg gtg     1680
ata gaa tac tgt gag aga gga tcc ctc cgg gaa gtt tta aat gac aca      1728
att tcc tac cct gat ggc aca ttc atg gat tgg gag ttt aag atc tct      1776
gtc ttg tat gac att gct aag gga atg tca tat ctg cac tcc agt aag      1824
aca gaa gtc cat ggt cgt ctg aaa tct acc aac tgc gta gtg gac agt      1872
aga atg gtg gtg aag atc act gat ttt ggc tgc aat tcc att tta cct      1920
cca aaa aag gac ctg tgg aca gct cca gag cac ctc cgc caa gcc aac      1968
atc tct cag aaa gga gat gtg tac agc tat ggg atc atc gca cag gag      2016
atc atc ctg cgg aaa gaa acc ttc tac act ttg agc tgt cgg gac cgg      2064
aat gag aag att ttc aga gtg gaa aat tcc aat gga atg aaa ccc ttc      2112
cgc cca gat tta ttc ttg gaa aca gca gag gaa aaa gag cta gaa gtg      2160
tac cta ctt gta aaa aac tgt tgg gag gaa gat cca gaa aag aga cca      2208
gat ttc aaa aaa att gag act aca ctt gcc aag ata ttt gga ctt ttt      2256
cat gac caa aaa aat gaa agc tat atg gat acc ttg atc cga cgt cta      2304
cag cta tat tct cga aac ctg gaa cat ctg gta gag gaa agg aca cag      2352
ctg tac aag gca gag agg gac agg gct gac aga ctt aac ttt atg ttg      2400
ctt cca agg cta gtg gta aag tct ctg aag gag aaa ggc ttt gtg gag      2448
ccg gaa cta tat gag gaa gtt aca atc tac ttc agt gac att gta ggt      2496
ttc act act atc tgc aaa tac agc acc ccc atg gaa gtg gtg gac atg      2544
ctt aat gac atc tat aag agt ttt gac cac att gtt gat cat cat gat      2592
gtc tac aag gtg gaa acc atc ggt gat gcg tac atg gtg gct agt ggt      2640
ttg cct aag aga aat ggc aat cgg cat gca ata gac att gcc aag atg      2688
gcc ttg gaa atc ctc agc ttc atg ggg acc ttt gag ctg gag cat ctt      2736
cct ggc ctc cca ata tgg att cgc att gga gtt cac tct ggt ccc tgt      2784
gct gct gga gtt gtg gga atc aag atg cct cgt tat tgt cta ttt gga      2832
gat acg gtc aac aca gcc tct agg atg gaa tcc act ggc ctc cct ttg      2880
aga att cac gtg agt ggc tcc acc ata gcc atc ctg aag aga act gag      2928
tgc cag ttc ctt tat gaa gtg aga gga gaa aca tac tta aag gga aga      2976
gga aat gag act acc tac tgg ctg  act ggg atg aag gac  cag aaa ttc      3024
aac ctg  cca acc cct cct act  gtg gag aat caa cag  cgt ttg caa      3069
gca gaa  ttt tca gac atg att  gcc aac tct tta cag  aaa aga cag      3114
gca gca  ggg ata aga agc caa  aaa ccc aga cgg gta  gcc agc tat      3159
aaa aaa  ggc act ctg gaa tac  ttg cag ctg aat acc  aca gac aag      3204
gag agc  acc tat ttt taa acctaaatga ggtataagga ctcacacaaa      3252
ttaaaataca gctgcactga ggcagcgacc tcaagtgtcc tgaaagctta cattttcctg      3312
agacctcaat gaagcagaaa tgtacttagg cttggctgcc ctgtctggaa catggacttt 3372
cttgcatgaa tcagatgtgt gttctcagtg aaataactac cttccactct ggaaccttat 3432
tccagcagtt gttccaggga gcttctacct ggaaaagaaa agaaatgaat agactatcta 3492
gaacttgaga agattttatt cttatttcat ttattttttg tttgtttatt tttatcgttt 3552
ttgtttactg gctttccttc tgtattcata agatttttta aattgtcata attatatttt 3612
aaatacccat cttcattaaa gtatatttaa ctcataattt ttgcagaaaa tatgctatat 3672
attaggcaag aataaaagct aaaggtttcc caaaaaaaaa a 3713

<210> 5
<211> 1073
<212> PRT
<213> homo sapiens

<400> 5

<210>  6
<211>  50
<212>  PRT
<213>  homo sapiens

<400>  6

Asp Arg Leu Asn Phe Met Leu Leu Pro Arg Leu Val Val Lys Ser Leu
1               5                   10                  15

Lys Glu Lys Gly Phe Val Glu Pro Glu Leu Tyr Glu Glu Val Thr Ile
            20                  25                  30

Tyr Phe Ser Asp Ile Val Gly Phe Thr Thr Ile Cys Lys Tyr Ser Thr
        35                  40                  45

Pro Met
    50

<210>  7
<211>  50
<212>  PRT
<213>  Pan troglodytes

<400>  7

Asp Arg Leu Asn Phe Met Leu Leu Pro Arg Leu Val Val Lys Ser Leu
1               5                   10                  15

Lys Glu Lys Gly Phe Val Glu Pro Glu Leu Tyr Glu Glu Val Thr Ile
            20                  25                  30

Tyr Phe Ser Asp Ile Val Gly Phe Thr Thr Ile Cys Lys Tyr Ser Thr
        35                  40                  45

Pro Met
    50

<210>  8
<211>  50
<212>  PRT
<213>  Canis lupus

<400>  8

Asp Arg Leu Asn Phe Met Leu Leu Pro Arg Leu Val Val Lys Ser Leu
1               5                   10                  15

Lys Glu Lys Gly Ile Val Glu Pro Glu Leu Tyr Glu Glu Val Thr Ile
            20                  25                  30

Tyr Phe Ser Asp Ile Val Gly Phe Thr Thr Ile Cys Lys Tyr Ser Thr
        35                  40                  45

Pro Met
    50

<210>  9
<211>  50
<212>  PRT
<213>  bos taurus

<400>  9

Asp Gln Leu Asn Phe Met Leu Leu Pro Arg Leu Val Val Lys Ser Leu
1               5                   10                  15

Lys Glu Lys Gly Phe Val Glu Pro Glu Leu Tyr Glu Glu Val Thr Val
            20                  25                  30

Tyr Phe Ser Asp Ile Val Gly Phe Thr Thr Ile Cys Lys Tyr Ser Thr
        35                  40                  45

Pro Met
    50

<210>  10
<211>  50
<212>  PRT
<213>  mus musculus

<400>  10

Asp His Leu Asn Phe Met Leu Leu Pro Arg Leu Val Val Lys Ser Leu
1               5                   10                  15

Lys Glu Lys Gly Ile Val Glu Pro Glu Leu Tyr Glu Glu Val Thr Ile
            20                  25                  30

Tyr Phe Ser Asp Ile Val Gly Phe Thr Thr Ile Cys Lys Tyr Ser Thr
        35                  40                  45

Pro Met
    50

<210>  11
<211>  50
<212>  PRT
<213>  rattus norvegicus

<400>  11

Asp His Leu Asn Phe Met Leu Leu Pro Arg Leu Val Val Lys Ser Leu
1               5                   10                  15

Lys Glu Lys Gly Ile Val Glu Pro Glu Leu Tyr Glu Glu Val Thr Ile
            20                  25                  30

Tyr Phe Ser Asp Ile Val Gly Phe Thr Thr Ile Cys Lys Tyr Ser Thr
        35                  40                  45

Pro Met
    50

<210>  12
<211>  50
<212>  PRT
<213>  gallus gallus

<400>  12

Asp Arg Leu Asn Phe Met Leu Leu Pro Arg Pro Val Val Lys Ser Leu
1               5                   10                  15

Lys Glu Thr Gly Leu Val Glu Pro Glu Phe Phe Glu Glu Val Thr Ile
            20                  25                  30

Tyr Phe Ser Asp Ile Val Gly Phe Thr Thr Leu Cys Lys Tyr Ser Thr
        35                  40                  45

Pro Met
    50

<210>  13
<211>  50
<212>  PRT
<213>  danio rerio

<400>  13

Asp Cys Leu Asn Phe Met Leu Leu Pro Gly Pro Val Val Arg Ser Leu
1               5                   10                  15

Lys Glu Thr Gly Lys Val Glu Pro Glu Leu Phe Asp Glu Val Thr Ile
            20                  25                  30

Tyr Phe Ser Asp Ile Val Gly Phe Thr Thr Leu Cys His Phe Ser Thr
        35                  40                  45

Pro Met

    50

## Description

The present invention relates in a first aspect to new nucleic acid molecules and peptides for identifying and diagnosing diarrhea syndrome including chronic diarrhea and bowel diseases. In addition, methods are provided for identifying or diagnosing a potential risk for developing or having a diarrhea syndrome comprising identifying in a sample from a subject the presence or absence of a mutation at position 2519 of the gene encoding GC-C. Moreover, a kit is provided allowing identification or diagnosing a diarrhea syndrome including probes allowing the detection of mutations at position 2519 of the nucleic acid sequence encoding GC-C or for detecting a mutation at position 840 of GC-C. Moreover, a method for screening and/or identifying candidate molecules useful in prophylactically or therapeutically treating said diseases is provided. Finally, the present invention relates to metformin and/or pharmaceutically acceptable salts thereof for use in the prophylactic or therapeutic treatment of diarrhea syndrome.

### Prior art

Diarrhea syndromes including acute and chronic diarrhea is a health problem which is diagnostically challenging and for which treatment is often ineffective. Often diarrhea syndromes, like acute or chronic diarrhea, are side effects of infections or exposure to toxins accompanying various disease and disorders. For example, Irritable bowel syndrome (IBS) affects 15-20% of adults, and is a common cause of diarrhea. Other causes include inflammatory bowel disease (IBD), infections, paraneoplastic hormones, celiac disease, malabsorption syndromes and bacterial overgrowth in the small intestine. In addition to organic causes, psychological factors have an important impact on bowel function.

Diarrhea is defined by the World Health Organization as having 3 or more loose or liquid stools per day, or as having more stools than is normal for that person.

### Different types of diarrhea have been defined:

### Secretory Diarrhea

Secretory diarrhea means that there is an increase in the active secretion, or there is an inhibition of absorption. There is little to no structural damage. The most common cause of this type of diarrhea is a cholera toxin that stimulates the secretion of anions, especially chloride ions. Therefore, to maintain a charge balance in the lumen, sodium is carried with it, along with water. In this type of diarrhea intestinal fluid secretion is isotonic with plasma even during fasting. It continues even when there is no oral food intake.

### Osmotic Diarrhea

Osmotic diarrhea occurs when too much water is drawn into the bowels. This can be the result of maldigestion (e.g., pancreatic disease or Coeliac disease), in which the nutrients are left in the lumen to pull in water. Osmotic diarrhea can also be caused by osmotic laxatives (which work to alleviate constipation by drawing water into the bowels). In healthy individuals, too much magnesium or vitamin C or undigested lactose can produce osmotic diarrhea and distention of the bowel. A person who has lactose intolerance can have difficulty absorbing lactose after an extraordinarily high intake of dairy products. In persons who have fructose malabsorption, excess fructose intake can also cause diarrhea. High-fructose foods that also have a high glucose content are more absorbable and less likely to cause diarrhea. Sugar alcohols such as sorbitol (often found in sugar-free foods) are difficult for the body to absorb and, in large amounts, may lead to osmotic diarrhea. Osmotic diarrhea stops when offending agent (e.g. milk, sorbitol) is stopped.

### Exudative Diarrhea

Exudative diarrhea occurs with the presence of blood and pus in the stool. This occurs with inflammatory bowel diseases, such as Crohn's disease or ulcerative colitis, and other severe infections such as *E. coli* or other forms of food poisoning.

### Motility-related Diarrhea

Motility-related diarrhea is caused by the rapid movement of food through the intestines (hypermotility). If the food moves too quickly through the gastrointestinal tract, there is not enough time for sufficient nutrients and water to be absorbed. This can be due to a vagotomy or diabetic neuropathy, or a complication of menstruation. Hyperthyroidism can produce hypermotility and lead to pseudodiarrhea and occasionally real diarrhea. Diarrhea can be treated with antimotility agents (such as loperamide). Hypermotility can be observed in people who have had portions of their bowel removed, allowing less total time for absorption of nutrients.

### Inflammatory Diarrhea

Inflammatory diarrhea occurs when there is damage to the mucosal lining or brush border, which leads to a passive loss of protein-rich fluids, and a decreased ability to absorb these lost fluids. Features of all three of the other types of diarrhea can be found in this type of diarrhea. It can be caused by bacterial infections, viral infections, parasitic infections, or autoimmune problems such as inflammatory bowel diseases. It can also be caused by tuberculosis, colon cancer, and enteritis.

Recent interest has focussed on the importance of genetic factors in the development of chronic diarrhea, as this may both provide insight into the pathophysiology of intestinal diseases and point to new treatments. IBS aggregates strongly in families and the genetic predisposition for Crohn's disease, particularly ileitis, is well documented (Franke A, et.al., Nat Genet 2010, 42(12):1118-25) but no major genes causing these disorders have been found. Both diseases are considered to be multifactorial and their etiology includes genetic susceptibility variants and environmental factors. Rare inherited forms of chronic diarrhea have been reported (Berni Canani R, et.al., J Pediatr Gastroenterol Nutr 2010, 50(4):360-6). Nearly all of these are severe autosomal recessive, single-gene diseases that manifest in the newborn period.

Thus, at present no diagnostic tool is available allowing identification of a predisposition for diarrhea syndrome including the above identified diseases, disorders or conditions. In addition, no marker or method is described allowing identification and screening for individual being at risk or suffering from diarrhea syndrome, including chronic diarrhea.

A fine balance exists between intestinal absorption and secretion of water and electrolytes. The secretory capacity of the small intestine is substantial, as demonstrated by the potentially life-threatening secretory diarrhea resulting from enterotoxigenic *E. coli* and *Vibrio cholera* infections. Heat-stable enterotoxin (ST) binds to intestinal receptor guanylate cyclase C (GC-C) resulting in elevated levels of cellular cyclic (c)GMP. cGMP elicits a signalling cascade involving protein kinases and the cystic fibrosis transmembrane conductance regulator (CFTR), ultimately causing increased chloride secretion through CFTR. This creates an osmotic drive that results in the secretion of Na⁺, and hence water, into the intestinal lumen, see e.g. Basu N, Arshad N, Visweswariah SS., Mol Cell Biochem 2010, 334(1-2):67-80, with further references.

In view of the above, there is an ongoing need for marker and methods allowing identification and diagnosis of diarrhea syndrome in individuals, in particular, for diagnosing chronic diarrhea or predisposition therefore.

### Brief description of the present invention

In a first aspect, the present invention relates to a nucleic acid molecule comprising the sequence of SEQ ID No. 1 or a nucleotide being complementary to said sequence. In particular, the nucleic acid molecule according to the present invention comprises the nucleic acid sequence of SEQ ID No. 2 having a T at position 2519.

In this connection, it is noted that the term "comprising" or "containing" as used herein includes the embodiment of "consisting of".

In a further aspect, a peptide is provided, said peptide being encoded by a nucleic acid molecule according to the present invention. In particular, the peptide is the guanylate-cyclase C (GC-C) having a mutation S840I at position 840 of SEQ ID No. 3.

In another embodiment, the present invention relates to a marker molecule for use in identifying or diagnosing diarrhea syndrome or predisposition for diarrhea syndrome. Said marker molecule allows the detection of a mutation at position 2519 of SEQ ID No. 4 or the mutation of S840I at position 840 of SEQ ID No. 5.

Moreover, a method for identifying or diagnosing a potential risk for developing, a predisposition for, or having a diarrhea syndrome in a subject is provided, comprising the step of analysing a biological sample from said subject by determining the presence or absence of a mutation at position 2519 of the sequence of SEQ ID No. 4 or a mutation at position 840 of SEQ ID No. 5 wherein the presence of such a mutation is correlated with said disease.

Furthermore, a kit for identifying or diagnosing a disease or the potential risk or predisposition for diarrhea syndrome is provided. Said kit encompasses at least one nucleic acid probe or oligonucleotide or at least one antibody determining the presence or absence of a mutation at position 2519 of the sequence of SEQ ID No. 4, in particular, G->T or a mutation at position 840 of SEQ ID No. 5, in particular, Ser -> Ile.

Another aspect of the present invention relates to a method for screening and/or identifying an agent useful in the prophylactic or therapeutic treatment of diarrhea syndrome including the step of determining whether a candidate agent can selectively modulate, in particular, decrease or inhibit the activity of GC-C.

A further aspect of the present invention relates to a new drug target useful in preventing or treating diarrhea syndrome whereby said drug target is GC-C.

Finally, the present invention provides new active ingredients and pharmaceutical compositions useful for the prophylactic or therapeutic treatment of diarrhea syndrome, in particular, of chronic diarrhea, in particular, of metformin and/or a pharmaceutically acceptable salt thereof.

### Brief description of the drawings

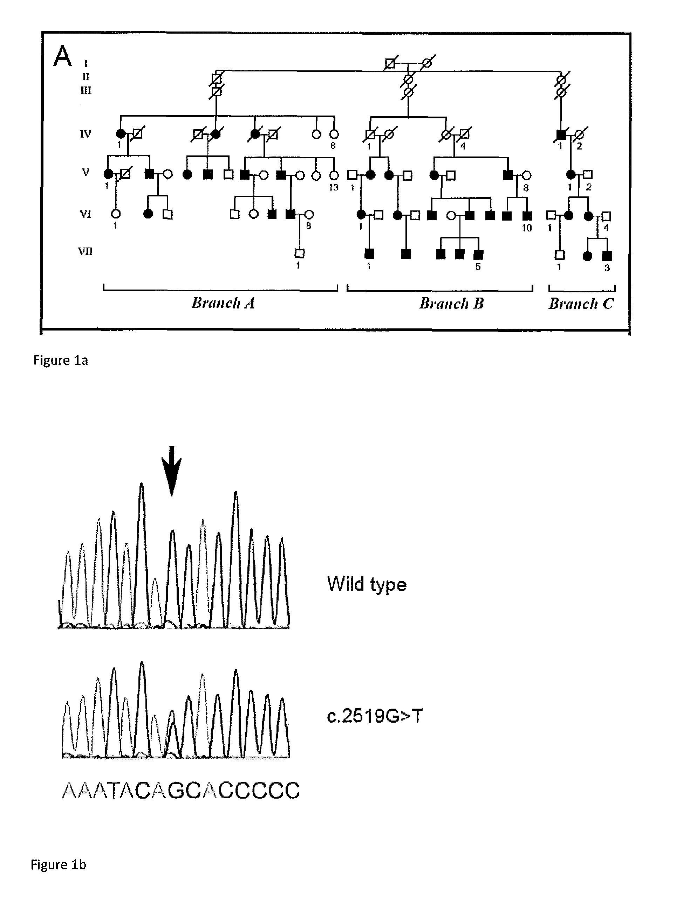

### Figure 1: Identification of a pathogenic mutation in a large family with chronic diarrhea.

Panel A shows the pedigree of the family. 18 affected males (black squares) and 14 affected females (black circles) were investigated. The inheritance pattern is autosomal dominant, and linkage analysis was performed using 14 healthy and 11 affected members of branch A. Number of generation is given in roman numbers to the left. Some numbers of individuals in each generation are also shown for each branch (from left to right). Information about disease status in diseased individuals in generation I-IV was limited, therefore most of these individuals are displayed as white squares/circles.

Panel B shows the heterozygous missense mutation (c.2519G>T, arrow) in exon 22 in the *GUCY2C* gene, which was found in all patients. This base change predicts the replacement of the amino acid serine in codon 840 with isoleucine [p.Ser840IIe] in the GC-C protein.

### Figure 2:

The region of the GC-C protein surrounding the amino acid serine (S, grey colour) in position 840 in the protein is displayed, with sequences from different species aligned. The high degree of conserved amino acids in the whole region may indicate that this region is important for protein function. Note residue tyrosine (Y) at position 820, which has been shown to be a site for inhibitory phosphorylation by c-src. The Ser840 (conserved in all compared species) is replaced by isoleucine in familial diarrhea syndrome patients.

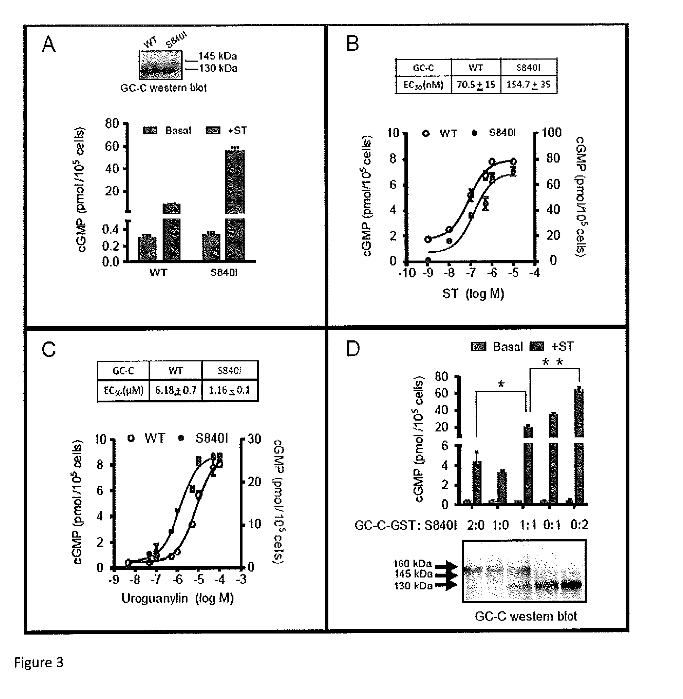

### Figure 3: Functional analyses of mutant GC-C-protein in HEK293T cells.

Panel A shows ST-mediated cGMP production by GC-C. HEK293T cells were transfected with plasmids that allowed expression of either WT GC-C or mutant GC-C S840I. Equivalent expression of WT or mutant GC-C was observed on Western blot analysis using a monoclonal antibody to GC-C (inset). GC-C migrates as two differentially glycosylated proteins of molecular weight 130 and 145 kDa. Cells were either treated with medium alone, or ST (10⁻⁷ M) and intracellular cGMP levels measured by radioimmunoassay.

Panel B shows ST dose response. Cells expressing either WT or mutant GC-C were treated for 15 min with varying concentrations of ST as indicated, and intracellular cGMP levels measured. Values shown are the mean ± SEM of a representative experiment with duplicate determinations. Experiments were repeated twice and EC₅₀ values were not significantly different (p < 0.07). Note different scales on the y-axes.

Panel C shows uroguanylin dose response. Cells expressing either WT or mutant GC-C were treated for 15 min with varying concentrations of uroguanylin as indicated, and intracellular cGMP levels measured. Values shown are the mean ± SEM of a representative experiment with duplicate determinations. Experiments were repeated twice and EC₅₀ values were significantly different (p < 0.001). Note different scales on the γ-axes.

Panel D shows heterodimerization of WT and mutant GC-C. Indicated ratios of plasmids expressing either WT GC-C tagged with glutathione S-transferase (GST) or mutant GC-C S840I were co-transfected in HEK293T cells. GC-C-GST migrates at a size of ~ 160 kDa due to the C-terminal fusion of GST, and mutant GC-CS8401 migrates at 130 and 145 kDa. Relative expression of the two proteins was monitored by Western blot analysis using a monoclonal antibody to GC-C. 72 h following transfection, cells were treated with ST (10⁻⁷ M) for 15 min and intracellular cGMP measured by radioimmunoassay. Data shown represent the mean ± SEM of duplicate determinations with experiments repeated twice (* p < 0.01; ** p < 0.0005).

### Figure 4:

The weekly average number of stools/day for patients treated with increasing doses of metformin (week 2-5) is shown. The patients were without medication in week 1, and then the dose of metformin was increased each week as shown by the horisontal bars, starting at the beginning of week 2 and stopping by the end of week 5. Patient identifiers are listed in bold letters in the panels. Patient A-V6 had a slower increase in dose due to slight kidney failure. Standard error of the mean are shown by error bars. (*): Statistically different number of stools/day compared to week 1 which preceded the treatment period (*t*-test, *p*<0.05). (1): Borderline statistical significant (*t*-test, *p*=0.055).

### Detailed description of the present invention

In a first aspect, the present invention relates to a nucleic acid molecule comprising the sequence of SEQ ID No. 1 or a nucleotide being complementary to the sequence of SEQ ID No. 1. It is preferred that the nucleic acid molecule according to the present invention is a nucleic acid molecule comprising, in particular, consisting of the sequence of SEQ ID No. 2 having a T at position 2519 (2519 G>T).

That is, the present inventors identified a new mutant form of GC-C in individuals, in particular, individuals suffering from diarrhea syndrome.

In a further aspect, the present invention relates to a peptide encoded by a nucleic acid molecule encoded by a nucleic acid molecule according to the present invention. In a preferred embodiment, said peptide according to the present invention is a guanylatcyclase C having a mutation S840I at position 840 of SEQ ID No. 3.

The above peptide or nucleic acid molecules are particularly useful as marker molecules for use in diagnosing or identifying individuals suffering from, having a predisposition for, or being at risk of developing diarrhea syndrome. Said marker molecules allow the detection of a mutation at position 2519 of SEQ ID No. 4, in particular, G->T, or allowing the detection of a mutation at position 840 of SEQ ID No.5, in particular, Ser -> Ile.

As used herein, the term diarrhea syndrome includes all types of diarrhea encompassing acute and chronic diarrhea. The diarrhea syndrome includes all types of diarrhea as described above.

In particular, the diarrhea syndrome is a secretory diarrhea including acute and chronic secretory diarrhea.

Further, the term "chronic diarrhea" includes diseases or conditions including Irritable bowel syndrome, Crohn's disease, Ulcerous colitis, Microscopic colitis, Coeliac disease, Hormonal and Carcinoid syndrome, VIPoma, Glucagonoma, Systemic Mastocytosis, Diabetic diarrhea.

Preferably, the diarrhea syndrome is a chronic secretory diarrhea or a acute secretory diarrhea.

As used herein, the term "GC-C" refers to the guanylate cyclise C including the wild type form, e.g. of Seq. ID. No. 5, as well as mutant forms and heterodimeric variants of wild-type and mutant forms unless otherwise indicated.

The marker molecule according to the present invention may be in a form of a suitable marker molecule known to the skilled person. Preferably, the marker molecule contains a label.

The label is preferably capable of producing, either directly or indirectly, a detectable signal. For example, the label may be radio-opaque or a radioisotope, such as ³H, ¹⁴C, ³²P, ³⁵S, ¹²³I, ¹²⁵I or ¹³¹I; a fluorescent (fluorophore) or chemiluminescent (chromophore) compound, such as fluorescein isothiocyanate, rhodamine or luciferin; an enzyme, such as alkaline phosphatase, beta-galactosidase or horseradish peroxidase; an imaging agent; magnetic or paramagnetic labels, or a metal ion.

In another embodiment, the detectable signal is detectable indirectly. For example, a labelled secondary antibody can be used to detect the protein of interest.

In another aspect, the present invention relates to a method for identifying or diagnosing a predisposition or potential risk for developing diarrhea syndrome or for identifying or diagnosing diarrhea syndrome in a subject. Said method comprises the step of analysing a biological sample from said subject for determining the presence or absence of a mutation at position 2519 of the sequence of SEQ ID No. 4 or a mutation at position 840 of SEQ ID No. 5 wherein the presence of such a mutation is correlated with said disease.

The terms "predisposed" or "predisposition" are used in the context of a probability that an individual may display clinical symptoms of diarrhea syndromes or that any existing, manifested clinical symptoms of diarrhea syndromes are the result of an underlying biochemical cause.

A "subject" in the context of the present invention is preferably a mammal. The mammal can be a human, non-human primate, mouse, rat, dog, cat, horse, or cow, but are not limited to these examples. A subject can be male or female. A subject can be one who has been previously diagnosed with or identified as suffering from or having a diarrhea syndrome. Alternatively, the subject may be one who has not been previously diagnosed or identified as being at risk or having a predisposition or suffering from or having a diarrhea syndrome.

In the context of the present invention, the term "biological sample" relates to a sample obtained from the subject including body fluid sample or tissue sample. The term "body fluid sample" or "sample of the body fluid" is a biological sample isolated from the subject which can include without being limited thereto, whole blood, serum, plasma, lymphatic fluids, ascetic fluids, interstitial fluids, cerebrospinal fluids, saliva, sputum, sweat, or any other secretion, excretion or other bodily fluids obtained from said individual. Preferably, the body fluid is blood, urine, or stool.

In a preferred embodiment of the method, a positive result for a mutation at position 2519 of SEQ ID No. 4 or position 840 of SEQ ID No. 5 is regarded as a positive indicator for a predisposition or potential risk, or for the diagnosis of said diseases.

That is, the present inventors recognised that a heterocygocity for the pathogenic human missense mutation 2519G to T in the GUCY2C-Gene, an intestinal receptor for bacterial heat-stable enterotoxins, is associated with diarrhea syndrome. The mutant receptor showed markedly increased cGMP production in response to its ligands compared to the wild-type receptor. It is assumed that this causes hyperactivation of the cystic fibrosis transmembrane regulator (CFTR) and, thus, increase chloride and water secretion from the enterocytes resulting in diarrhea syndrome, in particular, chronic secretory diarrhea.

It is preferred that the biological sample is a blood sample and that genomic DNA is analysed for the presence of the mutation according to the present invention. That is, in a preferred embodiment of the method according to the present invention, the test is based on detecting the mutation at nucleic acid level using common methods known to the person skilled in the art including using amplification methods, like PCR techniques, or, alternatively, using array techniques.

Alternatively, said method comprises the use of detecting the mutation at amino acid level. For example, antibodies, like monoclonal antibodies or antibody fragments, differentiating between the wild-type form wherein the amino acid serine is present at position 840 and the mutant peptide wherein the amino acid isoleucine is present at position 840. The skilled person is well aware of methods allowing the production of antibodies, in particular, monoclonal antibodies allowing differentiation between these two forms of GC-C. In addition, the skilled person is well aware of methods and techniques applied for analysing the biological sample for the presence or absence of a mutation at the prescribed position.

The term "antibody" as used herein is intended to include monoclonal antibodies, polyclonal antibodies, and chimeric antibodies. The antibody may be from recombinant sources and/or produced in transgenic animals. The term "antibody fragment" as used herein is intended to include Fab, Fab', F(ab')2, scFv, dsFv, ds-scFv, dimers, minibodies, diabodies, and multimers thereof and bispecific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bispecific antibody fragments and other fragments can also be synthesized by recombinant techniques.

Antibodies having specificity for the mutated form of GC-C may be prepared by conventional methods. A mammal, (e.g. a mouse, hamster, or rabbit) can be immunized with an immunogenic form of the peptide which elicits an antibody response in the mammal. Techniques for conferring immunogenicity on a peptide include conjugation to carriers or other techniques well known in the art. For example, the peptide can be administered in the presence of adjuvant. The progress of immunization can be monitored by detection of antibody titers in plasma or serum. Standard ELISA or other immunoassay procedures can be used with the immunogen as antigen to assess the levels of antibodies. Following immunization, antisera can be obtained and, if desired, polyclonal antibodies isolated from the sera.

In a preferred embodiment, the antibodies having specificity for the mutated form do not cross-react with non-mutated form.

In one embodiment of the invention, the agents, such as antibodies, antibody fragments, phage display proteins, aptamers, affibodies, chemical ligands or peptides that bind to GC-C, are labelled with a detectable label such as outlined above.

Furthermore, the present invention relates to a kit for identifying or diagnosing diarrhea syndrome or allowing the identification or determination of a predisposition, or a potential risk of diarrhea syndrome. Said kit comprises at least one nucleic acid probe or oligonucleotide or at least one antibody which can be used in a method according to the present invention for determining the presence or absence of a mutation at position 2519 of the sequence of SEQ ID No. 4 or a mutation at position 840 of SEQ ID No. 5. Optionally, the kit contains instructions for use in a method according to the present invention allowing determination of a predisposition for, or diagnosing diarrhea syndrome.

Preferably, the oligonucleotide present in the kit according to the present invention is at least one PCR primer, preferably, said PCR primer(s) allow the amplification of the nucleic acid of SEQ ID No. 1 or 2 or a fragment thereof.

Of course, the kit according to the present invention may contain additional components for determining the presence of the mutations according to the present invention. Said additional components include detection means including secondary labelled antibodies well known to the person skilled in the art or nucleic acid diction means like arrays, etc.

In still another aspect, the present invention relates to a method for screening and/or identifying an agent useful in prophylactically or therapeutically treating diarrhea syndrome, in particular, chronic or acute secretory diarrhea including the step of determining whether the candidate agent can selectively modulate the activity of GC-C. In particular, the candidate agent which may be useful in the prophylactic or therapeutic treatment of diarrhea syndrome is a compound inhibiting GC-C or reducing the activity of GC-C. It is preferred that the candidate agent is a compound inhibiting or reducing selectively GC-C. As demonstrated herein, the mutation identified in the present invention results in hyperactivation of GC-C. That is, the method may comprise screening based on the wild-type or the mutant GC-C as disclosed herein.

As used herein, "inhibition" in all its grammatical forms does not imply a complete cessation but rather indicates that the activity of GC-C being inhibited occurs at a lower rate or efficiency.

Further, as used herein, "prevention" in all its grammatical forms does not imply a complete prevention but rather indicates that the activity of GC-C or the clinical symptoms being prevented occurs at a lower rate or degree.

The candidate agent, which may be a prophylactic or therapeutic agent, according to the present invention is preferably selected from the group consisting of an isolated nucleic acid, a protein including polypeptides or oligopeptides, or a small molecule.

By "selective" or "selectively" is meant a candidate agent that primary affects expression or function of GC-C.

In a preferred embodiment, the method allows the production of a therapeutic agent for use in preventing or treating a diarrhea syndrome including chronic diarrhea, ileus, or bowel disease including inflammatory bowel disease or Crohn's disease.

In another aspect, the present invention relates to a drug target involved in diarrhea syndrome whereby said drug target is the guanylate cyclase C (GC-C). The GC-C include the wild type and, in particular, the mutant form of the present invention.

The drug targets represent druggable molecules, that is, molecules which allow the development of lead structures or drugs interacting therewith in order to inhibit function or expression thereof. In particular, these drug targets are drug targets for drugs of the group of small molecules.

Finally, the present invention relates to a metformin and/or a pharmaceutically acceptable salt thereof for use in the prophylactic or therapeutic treatment of diarrhea syndrome including chronic and acute secretory diarrhea, ileus, or bowel disease including inflammatory bowel disease or Crohn's disease.

That is, the present inventors recognised that the well known active metformin showing CFTR inhibiting activity reduce the symptoms of diarrhea syndrome, e.g. of chronic secretory diarrhea in affected individuals. Metformin and/or a pharmaceutical acceptable salt thereof are particularly useful for treating chronic secretory diarrhea.

Metformin is the international non-proprietary name of 1,1-dimethylbiguanide (CAS No. 657-24-9) also known as N,N-dimethylimidodicarbonimidic diamide. In the present invention, the term "metformin" includes any pharmaceutically acceptable salts thereof unless otherwise indicated. It is preferred that metformin is present in form of its hydrochloric salt. In addition, crystallic forms are included in the term "metformin".

Administration of metformin is not restricted. Typically metformin may be administered orally. The skilled person knows formulation of metformin suitable for treatment of diarrhea syndrome by oral administration. Metformin may be administered in suitable doses. In addition, metformin may be formulated in appropriated forms for administration. For example, for oral administration, metformin may be formulated in a solid dosage form, such as a tablet or pill.

That is, the active ingredients described herein may be in the form of pharmaceutically acceptable non-toxic salts thereof or other galenic preparations. Salts include acid added salts, such as salts with inorganic acids or with organic acids and also salts formed with cationic ions, like metallic ions, in particular alkali or alkaline earth metal or NH4+. Further, the active ingredients may be present in form of solvates thereof (e.g. hydrates).

In preferred embodiments, the pharmaceutical composition further comprises pharmaceutical additives or auxiliary substances, preferably, a pharmaceutically acceptable carrier, excipient or diluent.

The term "pharmaceutical composition" means a composition suitable for pharmaceutical use in a subject or individual, including an animal or human. A pharmaceutical composition generally comprises an effective amount of an active agent or ingredient and a carrier, including e.g. a pharmaceutically acceptable carrier.

A "therapeutic treatment" is a treatment administered to a subject or an individual to display symptoms or signs of pathology, disease, or disorder in which treatment is administered into the subject for the purpose of diminishing or eliminating those signs or symptoms of pathology, disease or disorder.

As used herein, the term "carrier" refers to a diluent, adjuvant, excipient or vehicle.

The composition comprising the compounds according to the present invention will be formulated, dosed, and administered in a fashion consistent with good medical practice. Factors for consideration in this context include the stage of the particular disease or disorder being treated, the particular mammal being treated, the clinical condition of the individual subject, the site of delivery of the agent, the method of administration, the scheduling of administration, and other factors known to medical practitioners. The effective amount of the agent to be administered will be governed by such considerations. The key factor in selecting an appropriate dose and scheduling is the result obtained, as indicated above. The compounds according to the present invention are administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated.

"Therapeutically- or pharmaceutically-effective amount" as applied to the compositions of the instant invention refers to the amount of composition sufficient to induce a desired biological result. That result can be alleviation of the signs and markers, symptoms, or causes of a disease, or any other desired alteration of a biological system.

In vitro assays as well as animal models may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition comprising the active ingredient according to the present invention in form of salts and solvates thereof to an individual.

The pharmaceutical composition according to the present invention comprises the active ingredient as described herein and, optionally, a pharmaceutical acceptable carrier. Such pharmaceutical compositions comprise a therapeutically effective amount of the active ingredient as defined herein and, optionally, a pharmaceutically acceptable carrier. The pharmaceutical composition may be administered with a physiologically acceptable carrier to a patient, as described herein. Acceptable means that the carrier be acceptable in the sense of being compatible with the other ingredients of the composition and not be deleterious to the recipient thereof. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, aerosols and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds, salts or solvates thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

The invention is further illustrated by the following examples.

### Materials and methods

### Patients

Thirty-two affected patients from three branches of the same family were studied (Fig.1A). All family members gave informed consent and were offered genetic counselling prior to the investigations. The healthy individuals were not subject to clinical investigation, whereas the affected individuals were all seen by a gastroenterologist and filled in questionnaires (Rome II and *ad hoc* forms) regarding bowel symptoms. The study was approved by the Regional Ethics committee of Western Norway, and the metformin treatment study also by the Norwegian Medicines Agency (Eudractnr. 2009-018071-13).

### DNA analyses and mutation detection

Whole genome SNP genotyping data are deposited in the Gene expression omnibus database (http://www.ncbi.nim.nih.gov/geo/), with accession number GSE31260. Link for reviewers is:
http://www.ncbi.nim.nih.gov/geo/query/acc.cgi?token=nvonzeeqomiqche&acc= GSE31260. PCR primers for amplification of exons and flanking intronic sequences of *GUCY2C* were designed using the Oligo 6.3 program (National Bioscience, Plymouth, USA). Reference sequence for *GUCY2C* was NM_004963.3. PCR amplification was performed using standard procedures.
DNA sequencing was performed using the ABI BigDye kit v1.1 and the ABI 3730 sequencer. Data analysis was assisted by use of the Seqscape software (ABI, Foster City, USA).

### Linkage analysis

Multi-point parametric linkage analysis and haplotyping was performed using the program Allegro v2 (Gudbjartsson D.F. et.al., Allegro version 2, Nat Genet 2005, 37:1015-6) on a set of 45000 SNPs pruned for strong local LD. Files were handled by the Uni Computing Linux cluster FIMM at the Bergen Center for Computational Science. We used a fully penetrant dominant inheritance mode with population disease allele frequency of 0.001. Marker allele frequencies were estimated using data from all individuals in the analysis. Physical marker positions are given according to NCBI Build 36.3 (http://www.ncbi.nlm.nih.gov/mapview/).

### Site directed mutagenesis

Generation of the c.2519G>T [p.Ser840IIe] mutant was performed as described earlier (Saha S., Biswas K.H., et.al., J. Biol. Chem. 2009, 284(40):27135-45). Wild type (WT) and mutant GC-C cDNAs were cloned in the mammalian expression vector pcDNA3 (Invitrogen, USA), and the respective proteins (GC-CWT and mutant GC-CS8401) were transiently expressed in HEK293T cells.

### Characterization of mutant protein GC-CS8401

Ligand-stimulated guanylate cyclase activity was measured in cells 72 h post-transfection, following addition of varying concentrations of ST or uroguanylin peptides for 15 min. Cells were then lysed in 0.1 N HCl and cGMP measured by radioimmunoassay as described earlier. Cells were also directly lysed in SDS sample buffer and subjected to Western blot analysis using a monoclonal antibody to GC-C. For *in vitro* guanylate cyclase assays, membranes were prepared from transfected cells (Ghanekar Y. et.al., Biochem J 2004, 379:653-63). Protein concentration was estimated by using a modification of the Bradford protein assay.

### Heterodimerization and interaction of WT and mutant GC-C

To monitor heterodimerization, plasmids harboring WT GC-C fused at the C-terminus to GST (GC-C-GST) as described in Saha S., et.al., J. Biol. Chem. 2009, 284(40):27135-45 and mutant GC-CS8401 were mixed in varying ratios for a total of 400 ng of DNA/well and co-transfected. In instances where only WT or mutant DNA (1:0 or 0:1) were to be transfected, vector control DNA was co-transfected to maintain the ratio of total DNA to lipid. 72 h post transfection, membrane protein (400 µg) was solubilized in interaction buffer (10 mM Tris-HCl pH 7.5, 5 µg/ml each of aprotinin, leupeptin and soybean trypsin inhibitor, 1% NP-40 and 5 mM β-mercaptoethanol), and solubilized protein incubated with 10 µL GSH beads for 1 h at 4 °C. Following washing of the beads, protein bound to the beads was subjected to Western blot analysis using the GC-C monoclonal antibody.

### Metformin treatment trial (I)

A pilot study (I) with metformin was performed in six individuals from branch A, with a low peroral dose of 500 mg twice daily. The trial lasted 10 weeks, and the patients registered number of stools (and texture) during week 1, 5 and 10. The first week was without treatment and the patients received 500 mg x 2 during weeks 2- 5. Weeks 6 - 10 were without treatment. Prior to starting the trial, all patients had a consultation with a gastroenterologist, with clinical examination, laboratory tests, ultrasound of abdomen and kidneys, colonoscopy with biopsies from colon (ascendens, transversum, sigmoideum) and terminal ileum. Number of stools per week was reduced by 40% in two patients with high stool frequency (35-36 per week). Three patients reported that stools were firmer during treatment.

**Table 2**

| **Patient** | **Sex/ age** | **Week 1 Stools per week** | **Week 5 Stools per week** | **Week 10 Stools per week** | **Week 5 Stool firmness** | **Side effects** |
|---|---|---|---|---|---|---|
| A-V1 | F 58 | 17 | 13 | 12 | | Intermittent nausea |
| A-V3 | M 62 | 17 | 18 | 14 | | None |
| A-V5 | F 58 | 23 | 20 | 21 | Increased | None |
| A-V6 | M 48 | 36 | 22 | 48 | | None |
| A-V10 | M 65 | 12 | 13 | 14 | Increased | None |
| A-V12 | F 26 | 35 | 20 | 30 | Increased | Increased bloating |

### Metformin treatment trial (II)

The dose given in the first metformin treatment trial (Table 2) was modest, and another pilot study with increasing metformin doses was started, including five individuals with high stool frequencies (22-42 per week). Patients with a history of ileus or inflammatory bowel disease were not included. Stool frequency was recorded continously for eight weeks. At day 1 of each week, blood samples were drawn for control of s-glucose, hemoglobin and creatinine. Symptoms and potential side-effects were followed closely by a weekly telephone call from the gastroenterologist.

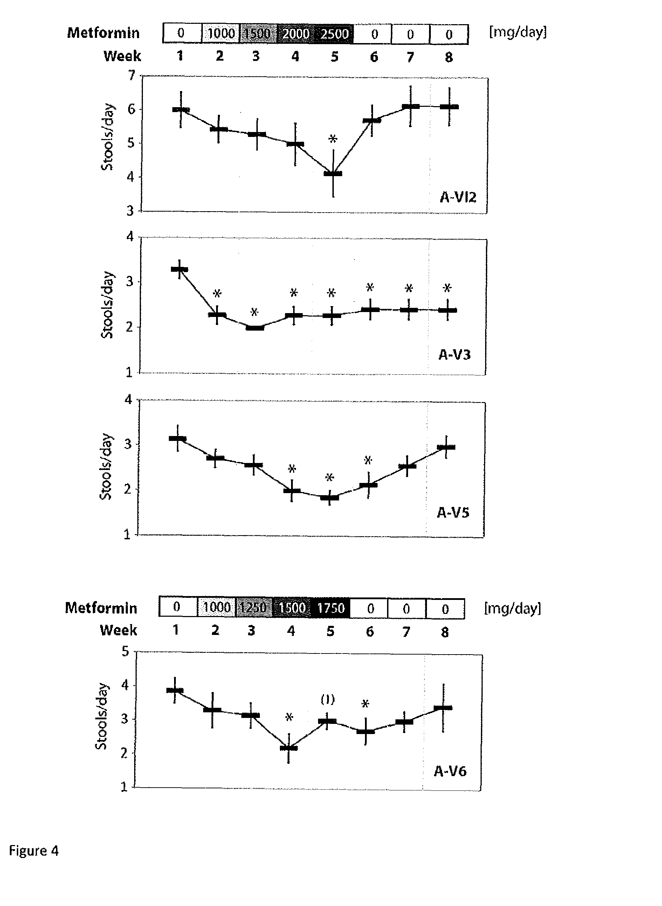

The weekly average number of stools/day for patients treated with increasing doses of metformin (week 2-5) is shown in Fig. 4. The patients were without medication in week 1, and then the dose of metformin was increased each week as shown by the horizontal bars, starting at the beginning of week 2 and stopping by the end of week 5. Patient identifiers are listed in bold red letters in the panels. Patient A-V6 had a slower increase in dose due to slight kidney failure.

### Results

### Clinical characterization

In the family, 32 affected individuals (14 women and 18 men) with a mean age of 43.8 years (range 2-89 years) were investigated. Family branch A came to the attention first, when the 88 year-old index person (A-IV1) was admitted to hospital due to dehydration (Fig. 1 A, Table 1). Those affected in branch A typically had a history of lifelong chronic diarrhea, starting in infancy, being fairly constant over the years, but tending to improve by middle age in some. Ten (of eleven) affected individuals in branch A had colonoscopies with no findings that could explain their condition (Table 1). The average number of stools per day was 3.6 (range 0.3-20) typically with watery or loose consistency, accompanied by meteorism and in some cases abdominal pain. The pattern of inheritance was autosomal dominant, with some variation in expression. Four cases (of 32) had previously been diagnosed as IBS (C-V12, B-V11, B-VI3, C-V113) but neither of these strictly fulfilled the Rome II criteria. However, five others did (A-IV1, A-V3, A-V16, B-V114, B-VII5).

With the subsequent inclusion of family branches B and C, more severe phenotypic aspects were revealed (Table 1). Ten patients had laparotomy for suspected bowel obstruction, of whom eight had mechanical ileus (including individual B-IV1 not shown in Table 1). Two patients with ileus had anatomical variants in the ileocecal region such as partly non-fixated ascending colon and slits in the ileal mesenterium. Three cases had early reoperation due to formation of adhesions, and cases C-V13 and B-IV1 were described as "adhesion"-ileus, even at their first laparotomy. Five affected with mechanical ileus eventually had resection of terminal ileum and in some cases also the cecum. Of these, four had verified or suspected Crohn's disease (Table 1). Additionally, patients B-VI8 and C-V12 were diagnosed with Crohn's disease, and patient A-V16 with possible eosinophilic enteritis. Crohn's disease was suspected in patient C-V1.

Eight patients were hospitalized with dehydration, metabolic acidosis and electrolyte disturbances already in the newborn period (Table 1). They had hyponatremia, hypokalemia and occasionally also hypomagnesemia and hypocalcemia, but none had verified infectious disease. This was accompanied by abdominal distension and dilatation of the small intestine. Hirschprung's disease was excluded in three patients including B-VII1, who at the age of 2 years exceptionally has constipation. Nine other patients were hospitalised with dehydration at various times later in life, frequently related to suspected or verified infectious gastroenteritis. The latter is reported by the affected to cause a longer period of recovery. Several other features recorded in our patients may be part of this inherited condition, including urolithiasis (four cases), vitamin B12 deficiency (six cases) and oesophagitis with or without oesophageal hernia (five cases). No evidence of behavioural disturbances or tendency to obesity or excessive leanness was detected in our patients. Most patients report food sensitivity, and several are careful with intake of fruit, vegetables and sweets.

### Detection of a possible causative mutation in the family

Linkage analysis using samples from 11 affected and 14 healthy individuals in family branch A revealed only one significant shared region in the affected on the short arm of chromosome 12 (12p), with a maximum LODscore of 5.1. A minimal haplotype spanning approximately 2.9 megabases (Mb) (at position 14,466,726-17,410,570 basepairs from start of 12p) showed complete cosegregation with the disease in the family. The region contained 28 putative protein coding genes. Among these, *GUCY2C* was sequenced first, since it encodes an intestinal transmembrane receptor guanylate cyclase with known function in ST-mediated diarrhea (Basu N., et al., Mol Cell Biochem 2010, 334(1-2):67-80). Sequencing of the entire coding region identified a heterozygous base substitution c.2519G>T in exon 22 (Fig. 1 B), predicting the replacement of the amino acid serine in codon 840 with isoleucine (p.Ser840IIe). This missense mutation was subsequently identified in all affected individuals in the family, and was not found in public databases (NCBI human SNPdb, build 132) or among 190 healthy local blood donors. None of the tested healthy individuals in the family had the mutation, i.e. we did not detect any cases of incomplete penetrance.

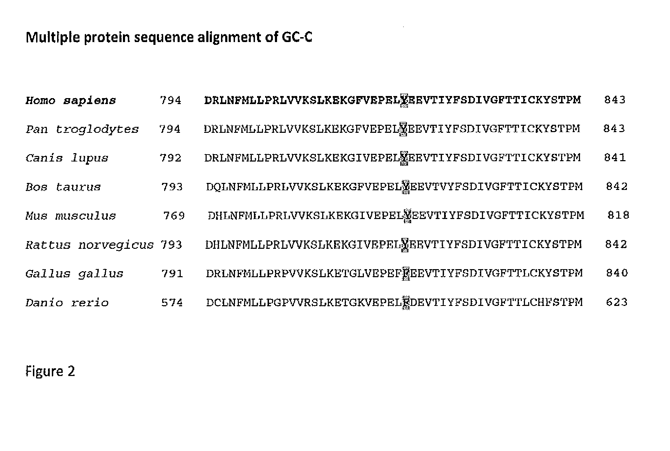

The amino acid Ser840 is highly conserved among mammalian GC-C proteins and also in chicken and zebrafish (Fig. 2). The substitution is located in the catalytic domain, and it is hypothesized that it may alter the guanylate cyclase activity of the mutant receptor. This was subsequently tested by heterologous expression in HEK293T cells *in vitro.*

### Functional characterisation of the p.Ser840IIe mutation

Experiments were performed both on intact cells (Fig. 3A-D) and on membranes prepared from transfected cells, expressing either wildtype (WT) or mutant (S840I) protein in equal amounts (Western blot, Fig. 3A). The basal GC-C enzyme activity and cellular cGMP-levels (Fig. 3A) and ligand (ST and uroguanylin) affinities were similar in cells transfected with either the WT or mutant receptor. However, both ST (Fig. 3A-B) and uroguanylin (Fig. 3C) activated the mutant receptor to a greater extent compared with WT receptor (note different scales for WT and mutant data). When cells were treated with 10⁻⁷ M ST (Fig. 3B) or 10⁻⁶ M uroguanylin (Fig. 3C), the mutant receptor showed 7-8 or 3-4 fold increases respectively in cGMP production, in comparison with the WT receptor. There was no significant difference in the EC₅₀ values for ST between mutant and WT receptors (Fig. 3B), in agreement with the observation that there was no difference in affinity of binding of ST. In contrast, the concentration of uroguanylin required for half maximal activation (EC₅₀) of mutant receptor was about six-fold lower than that of the WT receptor (Fig. 3C), indicating that uroguanylin acts more potently on the mutant receptor. This implies that the concentrations of uroguanylin present in the intestine could result in abnormally elevated levels of cellular cGMP in intestinal cells harboring the mutant receptor.

In patients who are heterozygous for the mutation, both WT and mutant receptors may be co-expressed in a single cell. In order to mimic the *in vivo* situation, WT receptor (as a fusion with GST) was co-expressed with the mutant receptor in HEK293T cells. Mutant receptor was pulled down by GSH beads along with the GST-tagged WT receptor, indicating their ability to form heterodimers. There was a significant increase (p value = 0.0057) in the production of cGMP when the two receptors were co-expressed (1:1) compared to expression of WT receptor (2:0, and 1:0) alone (Fig. 3D). This elevated activity was not as high as the activity seen with mutant receptor alone (0:1 and 0:2), indicating that heterodimerization of the WT and mutant receptor may reduce the ligand-mediated hyperactivation of the mutant receptor.

### Metformin treatment

The frequent watery stools and meteorism had been socially disabling for several of these patients, who so far had not been offered effective treatment. Hyperactivation of GC-C by ST has been convincingly demonstrated to stimulate CFTR and cause secretory diarrhea (Murek M. et al., Exp Physiol 2010, 95(4):471-8; Basu N., et al., Mol Cell Biochem 2010, 334(1-2):67-80). CFTR is inhibited by AMP-activated kinase (AMPK)-mediated phosphorylation. It has been tested if suppression of CFTR activity, through activation of AMPK with the common drug metformin (Zhou G., et.al., J Clin Invest 2001, 108(8):1167-74; McCarty M.F., et al., Med Hypotheses 2009, 73(6):1008-10), was sufficient to ameliorate patient symptoms. See the Table 2 (first trial with 1000 mg per day) and Fig. 4 (second trial, increasing dose) for details. There was a clear dose-response effect to metformin in four of five individuals, with maximal reduction of number of stools by 30-40% (Fig. 4).

There was a significant dose-response effect in four of five individuals, with reduction in weekly average stools/day by 30-40%. Gastrointestinal symptoms (meteorism, diarrhea, nausea) which are usually transient, are the most common complaints from patients starting with metformin.

**Table 1. Clinical characteristics of affected individuals.**

| All patients with exception of B-VI9 and B-VII1 report diarrhea or loose stools from neonatal age or childhood. This is combined with meteorism, frequently with very gaseous stools, and some have recurrent abdominal pain. Individuals marked * had worse diarrhea in childhood, and condition improved in adult age. ND: No data. | | | | | | | | | | | | |
|---|---|---|---|---|---|---|---|---|---|---|---|---|
| **Patient** | **Sex/ age** | **Stools Per day** | **Stool consistency** | **Meteorism** | **Recurrent abdominal pain** | **Hospitalisation due to electrolyte disturbances** | **Colonoscopy** | **Biopsies Colon/ ileum** | **Crohn's disease** | **Mechanical ileus** | **Resection of intestine** | **Dilated intestine** |
| A-IV1 | F 89 | 4-6 | Watery | ND | ND | Age 87 | Normal | Normal | - | - | - | Age 87 |
| A-IV4 | F 82 | 5-6 | Watery-explosive | ND | - | - | Normal | Normal | - | - | - | - |
| A-V1 | F 59 | 2-3* | Normal Intermittent diarrhea | + | - | Several times related to gastroenteritis in adult life | Normal | Normal | - | - | - | - |
| A-V3 | M 63 | 2-3 | Semisolid-watery | + | - | - | Normal | Normal | - | - | - | - |
| A-V5 | F 59 | 3-4 | Semisolid watery | + | - | - | Normal | Normal | | - | - | - |
| A-V6 | M 49 | 4-10 | Watery | ND | ND | - | 5 mm low grade adenoma. coecum | Normal | - | Suspected at age 48 | - | Age 48 |
| A-V8 | M 60 | 1-2* | Normal | + | - | - | ND | ND | - | - | - | - |
| A-V10 | M 67 | 2* | Semisolid | + | - | - | Diverticulum in terminal ileum | Normal | - | - | - | - |
| A-V12 | F 28 | 5-10 | Watery | ++ | - | - | Ulceration at the ileocecal valve | Granulation polyp | - | - | - | - |
| A-V16 | M 28 | 1-3 | Normal-semisolid, Intermittent diarrhea | + | + | - | Eosinophilic polyps | Eosinophilic cells | - | - | - | - |
| A-VI7 | M45 | 1-4 | Normal-semisolid Intermittent diarrhea | + | - | - | Normal | Normal | - | - | - | - |
| B-V2 | F 62 | 3-4 | Semisolid | +++ | + | Age 58 | Capsule endoscopy at age 60: Signs of Inflammatation in ileum, increasing in distal direction | Normal colon biopsies but biopsies of ileum not done at age 60 | Suspected at age 60 | 2x mechanical ileus aged 20 | 25 cm of terminal ileum | Grossly dilated ileum (10 cm) at surgery aged 20. Dilated ileum 4.4 cm at age 40 |
| B-V3 | F 65 | 3 | Watery-semisolid | +++ | + | ND | Normal | ND | - | Volvulus of coecum/colon ascendens aged 27. Adhesion-ileus 4 months later | - | Distended ileum at surgeries (10cm) |
| B-V5 | F 69 | 1-2* | Normal | + | Earlier not now | - | ND | ND | - | - | - | ND |
| B-V7 | M 73 | 1-3 | Normal to semisolid | + | + | - | ND | ND | - | ND | - | ND |
| B-VI1 | F 31 | 2-3 | Watery | +++ | - | 4 days old | Normal 1986 | ND | - | - | - | Dilated ileum 4,5 cm at 4 years |
| B-VI3 | F 40 | 4-12 | Watery | +++ | +++ | 2 weeks old | ND | ND | - | - | - | Distended abdomen and distended colon neonatal age |
| B-VI5 | M 45 | 4-6 | Watery | + | + | Neonatally | ND from colono- scopy at age 22. | Transmural ileal inflammation, focal active colitis at age 31 | Age 22 | Age 31^{a} | 15cm ileum and 5 cm coecum, then 170cm of ileum | Gross dilatation at surgery |
| 8-V17 | M 43 | 6-10 | Watery | +++ | +++ | - | ND | Transmural ileal inflammation, no granulomas at age 38 | Age 38 | Age 38 and 39 | Ileal resection both at age 38 and 39.^{b} | Now permanent gross dilatation (10 cm) in distal 1m of ileum |
| B-VI8 | M 38 | 0-4 | Watery-semisolid | + | + | Age 17months, convulsions | Colitis and apthous lesions all segments. terminal ileitis age 37 | Active colons all segments, mild ileitis age 37 | Age 37 | - | - | Moderately dilated ileum on MRI |
| B-VI9 | M 41 | 2-5 | Diarrhea, watery- semisolid, started at age 15^{c} | - | - | After gastroenteritis at age 35 | Ileal inflammation first noticed at age 30, also at age 34 and 41 | Ileal inflammation. No granulomas. | Age 30 | Age 35: Stenotic terminal ileum | Resection of 50 cm of terminal ileum | Dilated ileum proximal to a narrow terminal ileum at age 30 |
| B-VI10 | M 44 | 1-2 | Intermittent diarrhea | + | - | ND | ND | ND | ND | ND | ND | ND |
| B-WII1 | M 2 | 1-2 *per week* | Neonatal diarrhea, *but now constipation* | ND | ND | 3 weeks old | ND | Rectal biopsies normal | - | - | - | Dilated intestinal loops neonatally |
| B-VII2 | M 3 | 2-3 | Watery-semisolid | + | ND | 6months 2 ½ years | Normal | Normal | - | Age 6 months: Suspected sigmoideum volvulus related to rotavirus infection | - | Dilated colon at surgery aged 6 months |
| 8-VII3 | M 17 | 5 | Watery | +++ | +++ | 1 month | ND | ND | - | Age 10: Distal ileum rotated and hemiated through mesenterial slit | - | Dilated 30-40 cm of distal ileum at surgery |
| B-VII4 | M 16 | 1-3 | Normal-semisolid | +++ | +++ | Neonatally Several times later related to gastroenteritis | ND | ND | - | - | - | Dilated rectum, 9 cm |
| 8-VII5 | M 12 | 2-5 | Watery-semisolid | +++ | + | Neonatally | ND | ND | - | - | - | - |
| C-V1 | F 65 | 10-20 | Watery | ++ | ++ | - | ND | ND | Suspected at age 65, high calprotectin d | - | - | Dilated distal ileum (4 cm), air-fluid-levels on X-ray |
| C-VI2 | F 44 | 4-5 | Watery-semisolid | +++ | +++ | - | Ileal apths, sigmoideal inflammation aged 41 | Sigmoidal inflammation | Suspected at age 41. At present untreated (no inflammation) | Suspected at age 32 | - | Atonic dilated ileum at explorative laparotomy age 32 |
| C-VI3 | F 41 | 4-72 | Watery-semisolid | +++ | +++ | - | ND | ND | - | Mechanical ileus aged 41^{c} | - | Grossly dilated ileum at laparotomy |
| C-VII2 | F4 | >3 | Watery-semisolid | + | + | Neonatally | ND | ND | - | - | - | - |
| C VII3 | M17 | 3-4 | Watery, occasional formed stools | +++ | +++ | In school age | Normal | ND | - | - | - | - |

| | | | | | | | | | | | | |
|---|---|---|---|---|---|---|---|---|---|---|---|---|
| ^{a} Termial ileitis with 13 cm long ulcer at age 31. First resection of 15cm ileum and 5 cm cecum. Reoperation 7 days later due to ileus. 5 weeks later again ileus and 170cm intestine was resected. ^{b} Resection of 12 cm terminal ileum at age 38 and 30cm of terminal ileum at age 39. At age 39 there was no stenosis, but an atonic seg ment of ileum. ^{c} Diarrhea started at age 5 after a gastroenteritis. Diarrhea subsided in his twenties only to reappear permanently from age 28. ^{d} Calprotectin measured recently was 243 mg/kg feces, which may indicate inflammation. Mucus and occasionally blood has been observed In feces. The patient also had hypomagnesemia and hypokalemia. ^{c} Ileus of distal ileum described as caused by adhesions (first laparotomy). New signs of ileus and new laparotomy 7 days later, gross dilatation of distal ileum (7 cm), but intestine was viable and resection was not performed. Patient experienced large fluid losses. | | | | | | | | | | | | |

## Claims

1. A nucleic acid molecule comprising the sequence of SEQ ID. No. 1 or a nucleic acid moleculle being complementary to the sequence of SEQ ID No. 1.

2. The nucleic acid molecule of claim 1, comprising, in particular, consisting of, the sequence of SEQ ID No. 2 having a T at position 2519.

3. A peptide encoded by a nucleic acid molecule according to claim 1 or 2.

4. The peptide of claim 3 being guanylate cyclase C (GC-C) having a mutation S840I at position 840 of SEQ ID No. 3.

5. Marker molecule for use in diagnosing diarrhea syndrome, like bowel diseases, including inflammatory bowel disease and Crohn's disease, ileus, in particular, of acute or chronic secretory diarrhea allowing for the detection of a mutation at position 2519 of SEQ ID No. 4, preferably comprising, in particular, consisting of, the nucleic acid molecules or peptides as identified in any one of claims 1 to 4 or its complementary sequence.

6. The marker molecule of claim 5 for use in diagnosing chronic or acute secretory diarrhea.

7. A method for identifying or diagnosing a potential risk for developing or having a diarrhea syndrome including a disease of chronic or acute diarrhea, ileus, bowel disease including inflammatory bowel disease or Crohn's disease, in a subject which comprises step of analyzing a biological sample from said subject by determining the presence or absence of a mutation at position 2519 of the sequence of SEQ ID No. 4 or a mutation at position 840 of SEQ ID No. 5 wherein the presence of such a mutation is correlated with said disease.

8. The method according claim 7 where a positive result for a mutation at Pos. 2519 of SEQ ID No. 4 or Pos. 840 of SEQ ID No. 5 is regarded as a positive indicator for a potential risk or for diagnosing the disease.

9. A method according claim 7 or 8, **characterized in that** genomic DNA, preferably obtained from a blood sample, is used for identifying the mutation.

10. The method according to any one of claims 7 to 9 wherein the test is based on detecting the mutation at nucleic acid level using amplification methods, like PCR techniques or, alternatively, using array techniques.

11. A kit for identifying or diagnosing a disease or the potential risk for a disease of diarrhea syndrome including chronic or acute secretory diarrhea, ileus, or bowel disease including inflammatory bowel disease or Crohn's disease in a subject comprising at least one nucleic acid probe or oligonucleotide or at least one antibody, which can be used in a method as defined in any one of claims 7 to 10 for determining the presence or absence of a mutation at position 2519 of the sequence of SEQ ID No. 4 or a mutation at position 840 of SEQ ID No. 5.

12. The kit according to claim 11 wherein said oligonucleotide is at least one PCR primer, preferably a set of PCR primers, allowing the amplification of the nucleic acid of SEQ ID No. 1 or 2 or a fragment thereof.

13. A method for screening and/or identifying an agent useful in prophylactically or therapeutically treating a disease of diarrhea syndrome, including chronic or acute secretory diarrhea, bowel disease or Crohn's disease including the step of determining whether a candidate agent can selectively modulate the activity of GC-C, in particular, the mutant GC-C of Seq. ID. No. 3.

14. The method according to claim 13 for producing a therapeutic agent for use in preventing or treating diarrhea syndrome including a disease of chronic or acute secretory diarrhea, ileus, bowel disease including inflammatory bowel disease or Crohn's disease.

15. A drug target for the treatment of a disease of diarrhea syndrome including chronic or acute secretory diarrhea, ileus bowel disease including inflammatory bowel disease or Crohn's disease which is GC-C, in particular, the mutant GC-C of Seq. ID. No.3.

16. Metformin and/or a pharmaceutically acceptable salt thereof for use in the prophylactic or therapeutic treatment of a disease of diarrhea syndrome including chronic or acute secretory diarrhea, ileus, bowel disease including inflammatory bowel disease or Crohn's disease.

17. Metformin and/or a pharmaceutically accaptable salt thereof according to claim 16 for use in treating chronic or acute secretory diarrhea.
